# EUROPEAN PATENT SPECIFICATION

(11) **EP 4 057 938 B1**
(45) Date of publication and mention of the grant of the patent: **08.07.2026**
(21) Application number: 19952393.7
(22) Date of filing: 31.12.2019
(51) Int. Cl.: A61F 2/00, A61L 31/04, A61L 31/10, A61L 31/14, C08G 63/08

(54) **A METHOD OF MANUFACTURING A MESH PROSTHESIS**
VERFAHREN ZUR HERSTELLUNG EINER NETZPROTHESE
PROCÉDÉ DE FABRICATION D'UNE PROTHÈSE EN TREILLIS

(30) Priority: 12.11.2019 IN 201921045955
(43) Date of publication of application: 21.09.2022
(73) Proprietor: Meril Endosurgery Pvt. Ltd., Vapi, Gujarat 396191 (IN)
(72) Inventor: MINOCHA, Pramod Kumar, Vapi- 396191, Gujarat Vapi (IN); KOTHWALA, Deveshkumar Mahendralal, Surat-395003, Gujarat (IN); BHATT, Vibhuti Shailesh, Vapi, Gujarat Vapi 396195 (IN)
(74) Representative: Marks & Clerk LLP
(86) International application number: PCT/IN2019/050967
(87) International publication number: WO 2021/095045

(56) References cited:
- US-A1- 2010 318 108
- US-A1- 2010 318 108
- US-B2- 10 350 045
- US-B2- 10 350 045
- US-B2- 10 433 942
- US-B2- 10 433 942

## Description

### FIELD OF INVENTION

The present invention relates to a method of manufacturing a mesh prosthesis.

### BACKGROUND

Hernia is a condition which occurs when a part of an organ is displaced and protrudes through the walls of the cavity including the said organ. In some cases, hernia may be visible as an external bulge particularly when a person is straining or bearing down. Hernia may occur in different parts of the body based on which hernia may be of different types such as, abdominal hernia, hiatus hernia, incisional hernia and umbilical hernia. Conventionally, hernia was treated using procedures such as direct suture and tissue repair in terms of recurrence. However, the said conventional procedures were not effective and posed harmful consequences when performed on a patient.

In order to overcome the problems posed by the aforesaid procedures, a new surgical procedure which is utilized to treat hernia known as the hernia repair procedure was introduced. The aforesaid surgical procedure is performed using artificial, alloplastic meshes. The use of the said meshes to heal hernia provided better results however, the said meshes had high recurrence rates due to the low stretching capability of the mesh/tissue and complex contrasts to highly elastic abdominal walls inside the body of the patient.

To overcome the above mentioned problem, the mesh prosthesis was provided with a layer of film. The said hernia film prosthesis when attached on an intraperitoneal region of a patient produces adhesion which provides better sticking properties. Due to the efficient sticking property of the film, the film provides better support to the abnormal part of the viscous organ (i.e. the part of the body affected with the hernia).

The design and the implantation technique of the mesh prosthesis depend on the different types of hernia (as described above). For example, a natural polymer coated mesh is used for abdominal hernia. However, the natural polymer coated mesh is unable to retain its characteristic shape or physical function. Hence, synthetic polymers meshes are utilized in order to overcome the problems caused by the natural tissue meshes.

For instance, the US Patent 20160242891A1 discloses an absorbable membrane wherein the absorbable membrane is prepared with a mixed solution of PLCL and MPEG-PLLA copolymer and a non-absorbable polypropylene mesh. According to the method disclosed in the said patent, the absorbable membrane is attached with the non-absorbable membrane through a press fit method. The said method may lead to non-uniform adherence of the absorbable membrane to the polypropylene mesh. Furthermore, the press-fitted polypropylene mesh with absorbable membrane is only subjected to static evaporation which may leave residual solvents within the repair material.

US2010/318108A1 discloses a method for manufacturing a composite implantable device. The method includes preparing a biodurable reticulated elastomeric matrix having a three-dimensional porous structure comprising a continuous network of interconnected and intercommunicating open pores, applying an adhesive to a polymeric surgical mesh, and affixing the mesh to a face of the matrix to form the composite implantable device. The mesh comprises a plurality of intersecting one-dimensional reinforcement elements.

US10350045B2 discloses a method for preparing a repair material with multi-purpose for an abdominal wall hernia. The method comprises steps of: (1) immersing a polypropylene mesh into a mixed solution for forming an absorbable coating on a surface of the polypropylene mesh after being dried; (2) pouring the mixed solution into a mould, statically evaporating for obtaining an absorbable membrane; and (3) press-fitting the polypropylene mesh obtained in the step (1) to the absorbable membrane and statically evaporating; wherein the mixed solution is a mixed solution of a PLCL and an MPEG-PLLA copolymer.

US10433942B2 disclosed the surgical implant comprising an areal, flexible, mesh-like basic structure including a knitted fabric made in gallon crotcheting technique. In this fabric, mesh pores are formed from chains (generally in warp direction) made in closed pillar stitch. Adjacent chains are connected to each other, by at least one inlaying weft thread, in sections forming a respective common side of two adjacent mesh pores. The inlaying weft thread, in the region of said common side, runs in at least three loop courses.

Therefore, a mesh prosthesis which overcomes the drawbacks of the conventional assemblies is required to be devised.

### SUMMARY

The present invention relates to a method for manufacturing a mesh prosthesis as set forth in the appended claims. The said method includes solvent casting of a polymeric solution in a substrate. The polymeric solution is poured inside the substrate to commence the process of solvent casting in a way that there is no inadvertent introduction of air bubbles.

Post the solvent casting method, the substrate is subjected to a primary annealing process. The said method is performed in order to evaporate a partial amount of solvent from the substrate. The step of primary annealing is performed at a predefined temperature and for a predefined time period. The primary annealing solution is performed to form a uniform thin film layer. The film layer formed during the said step is separated from the substrate.

After the formation of the film layer via the primary annealing process, the film layer is placed in close contact with a mesh layer. The said placement may be carried out via adhesion of the film layer with the mesh layer. In an embodiment, the adhesion process is performed with the help of an adhesive agent. Post adhesion, the film layer is compressed with the mesh layer in order to form a mesh prosthesis. The compression is performed such that even force is applied to the mesh layer throughout its surface.

Post adhesion and compression, the process of secondary annealing is performed on the mesh prosthesis. The said process is performed to ensure complete and uniform adhesion and compression of the film layer with the mesh layer. The process of secondary annealing is performed at a predefined temperature and for a predefined time period. The secondary annealing process relieves any internal stresses generated in the mesh prosthesis and further enhances the strength of the film layer.

Post the secondary annealing process, the annealed mesh prosthesis is incubated with an alcohol in order to remove excessive moisture that may be present in the annealed mesh prosthesis. The alcohol incubated mesh prosthesis results into a completely dry prosthesis that easily unfolds at an implantation site during the deployment process.

The mesh prosthesis is further subjected to the formation of drainage holes. The drainage holes are formed in the mesh layer of the mesh prosthesis in order to facilitate fibroblast growth and to ensure drainage of seroma. In an embodiment, the drainage holes are formed using laser technique.

The mesh prosthesis is further subjected to the formation of an indicator on a centre portion of the film layer. The indicator is provided in the form of an alphabet. In an embodiment, the indicator is formed using the solvent casting method. The said indicator helps in identifying the visceral side of the mesh prosthesis as well the orientation of the mesh prosthesis from the centre portion of the said prosthesis.

Post the formation of the indicator, the mesh prosthesis is subjected to packaging and sterilization.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein. Moreover, those in the art will understand that the drawings are not to scale.
Fig. 1 depicts a schematic view of the mesh prosthesis in accordance with an embodiment of the present invention.
Fig. 1a depicts a back view of the mesh prosthesis in accordance with an embodiment of the present invention.
Fig. 2 illustrates a flow chart depicting the method of manufacturing the mesh prosthesis in accordance with an embodiment of the present invention.

### DETAILED DESCRIPTION OF THE DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Particular embodiments of the present disclosure are described herein below with reference to the accompanying drawings, however, it is to be understood that the disclosed embodiments are merely examples of the disclosure, which may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In accordance with the present disclosure, mesh prosthesis and a method of manufacturing thereof are disclosed. The mesh prosthesis of the present invention is utilized for treatment of hernia in particular, abdominal wall hernia. Although the mesh prosthesis is utilized for the treatment of hernia, the said prosthesis may also be utilized for the treatment of other body organs. The mesh prosthesis of the present invention is a multi-layered prosthesis for example, prosthesis with a first layer and a second layer. The first layer is a non-absorbable mesh layer (i.e. the mesh layer) while the second layer is an absorbable layer provided in the form of a film (i.e. the film layer). The second layer of the mesh prosthesis further includes an indicator in the form of an alphabet. The indicator is provided for example, at a center portion of the mesh prosthesis. The said indicator may be visible from a front surface as well as from a back surface of the mesh prosthesis. The indicator may be colored for easy identification when the said indicator is disposed on the film layer (i.e. the second layer) of the mesh prosthesis. The indicator is used to identify the visceral side of the mesh prosthesis and also ensures the ease of mesh orientation from the center of the mesh prosthesis.

The method for preparing the mesh prosthesis of the present invention includes the steps of primary annealing and secondary annealing. The said steps ensure firm attachment of the mesh layer (first layer) with the film layer (second layer) of mesh prosthesis. The said steps also ensure uniform adhesion of the film layer to the mesh layer and the transparency of the film layer.

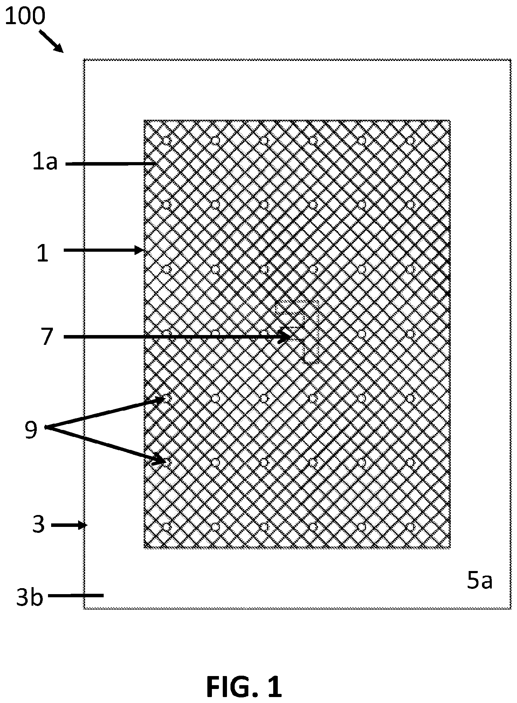

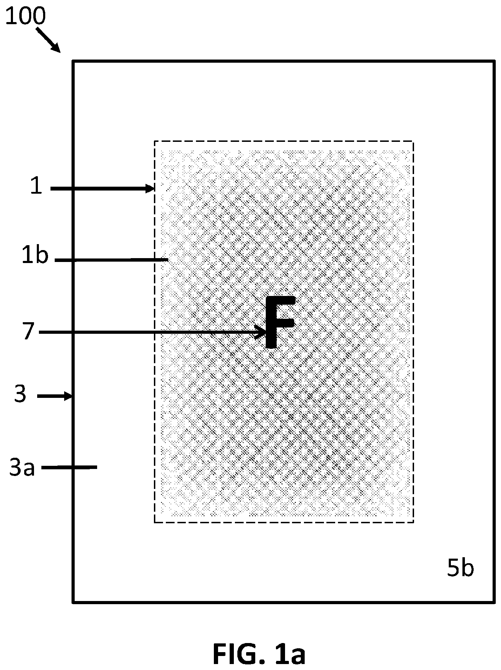

Now specifically referring to the drawings, Figs. 1 and 1a depict the schematic views of the mesh prosthesis 100 of the present invention. The shape of the mesh prosthesis 100 may be without limitation circular, rectangular or any shape known in the art. In an embodiment, the mesh prosthesis 100 is rectangular in shape. As depicted in the Figs. 1 and 1a, the mesh prosthesis 100 may include without limitation a mesh layer 1, a film layer 3, a front surface 5a, a back surface 5b (as depicted in FIG. 1a), an indicator 7 and drainage holes 9.

In an embodiment, the mesh prosthesis 100 of the present invention may be dual layered mesh. The dual layered mesh prosthesis 100 includes- the mesh layer 1 and the film layer 3.

The mesh layer 1 may be made from a non-absorbable material. The non-absorbable material may include without limitation synthetic polymers such as, polypropylene, polytetrafluroethylene (PTFE), dacron, orlon, polyethylene, mylar or marlex. In an embodiment, the mesh layer 1 is made of synthetic polymer polypropylene. The mesh layer 1 is made of polypropylene due to its excellent durability, high tensile strength and low infection risk and comfortable for patients.

The mesh layer 1 may be formed by knitting one or more strands of the synthetic polymer with one another. The mesh layer 1 may be knitted using any knitting pattern known in the art. The mesh layer 1 is knitted in a manner such that the pore size of the mesh layer 1 may range from 0.5- 5.0mm more preferably, from 1.0- 2.0mm. In an embodiment, the mesh layer 1 has burst strength in the range of 06 -15 kgf and is sufficiently flexible.

The mesh layer 1 includes a bottom surface 1a and a top surface 1b (shown in FIG. 1a). The bottom surface 1a of the mesh layer 1 may be provided with a plurality of drainage holes 9. The drainage holes 9 are disposed over the mesh layer 1 in order to facilitate the fibroblast growth. The said holes also ensure the drainage of seroma from the patient's body in a predefined time period.

The diameter of the drainage holes 9 may depend on the pore size of the mesh layer 1. In an embodiment, the diameter of the drainage holes 9 must be less than the pore size of the mesh layer 1. As the pore size of the mesh layer 1 is in the range of 1.5 - 2.5 mm, the diameter of the drainage hole 9 is in the range of 0.5- 1.25 mm.

The film layer 3 may be made from an absorbable material. The absorbable material may include without limitation, a polymer or a blend of polymers. In an embodiment, poly-L-lactide-co-caprolactone (PLCL) is utilized for making the film layer 3 due to its mechanical properties. PLCL is flexible and durable material which mirrors the chemical properties of its monomers including lactide and caprolactone. The lactide monomer in PLCL provides toughness and the caprolactone monomer has an additional chain of carbohydrates which enhances elasticity of the film layer 3. PLCL is an absorbable polymer that degrades in a patient's body in about twelve months through the body's natural metabolism which turns into carbon dioxide and water. The ratio of L-lactide: caprolactone in PLCL may range from 95:05, 85:15 more preferably, 70:30. In an embodiment, the ratio of L-lactide: caprolactone in PLCL is 70% L-lactide and 30% of caprolactone.

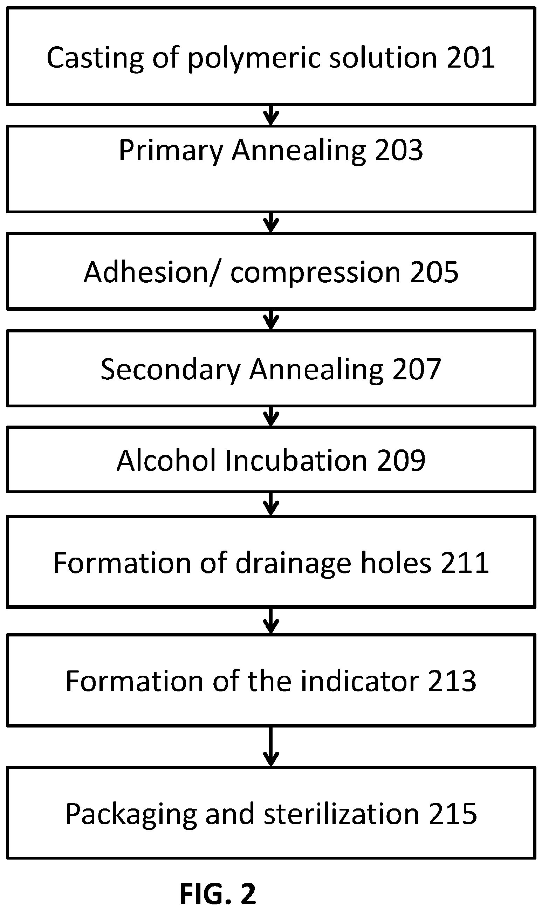

The film layer 3 may be prepared by using various techniques known in the art. The said techniques may include without limitation, solvent casting and hot melt extrusion. In an embodiment, the technique used to prepare the film layer 3 is solvent casting (described in detail in Fig. 2). In an embodiment, the film layer 3 formed using the solvent casting method is a transparent film layer.

The solvent casting method is used for preparing the film layer 3 as the said method includes a simplified manufacturing process and a low cost of processing the film layer 3. The solvent casting method requires thorough analysis of the rheological properties of the polymers utilized to prepare the film layer 3. The rheological properties of the polymers used affect the drying rate, film thickness and uniformity of films formed to make the film layer 3.

The solvent casting method is performed using a polymeric solution including the polymer (such as PLCL) with one or more solvents. The one more solvents are selected as per the solubility properties of the polymers used to make the film layer 3. The one or more solvents may include without limitation benzyl alcohol, chloroform, acetone, acetonitrile, dichloromethane and mixtures thereof. In an embodiment, the solvent used to formulate the film layer 3 is dichloromethane. The concentration of the polymeric solution utilized to make the film layer 3 may range from 1.0% - 5.0% (w/v) more preferably, 2.0% - 4.0% (w/v). In an embodiment, the concentration of the polymeric solution is 3.0 % to 3.5 % (w/v). The viscosity of the polymeric solution ranges from 0.1- 1.5 dl/gm more preferably, 0.6 - 0.9 dl/gm. In an embodiment, the viscosity of the polymeric solution is 0.6 - 0.9dl/gm. The viscosity of the polymeric solution acts as an important parameter during the solvent casting method for the formation of the film layer 3 as variation in viscosity of the polymeric solution may affect the rate of drying of the film layer 3.

Optionally/additionally, the film layer 3 may also include one or more non-active ingredients such as, plasticizers. The plasticizers may include without limitation, triethyl citrate, tributyl citrate, acetyl tributyl citrate (ATBC), acetyl triethyl citrate, glycerol, polyethylene glycols, methoxypolyethylene glycols and likewise. In an embodiment, the plasticizer used in the formation of the film layer 3 is acetyl tributyl citrate. The use of plasticizer helps in reducing the brittleness of the polymer film formed while promoting its flexibility and plasticity for the film layer 3.

The film layer 3 may also include a top surface 3a (as shown in FIG. 1a) and a bottom surface 3b. The bottom surface 3b of the film layer 3 may be attached to the top surface 1b of the mesh layer 1 via the annealing process (described in detail in Fig. 2) in order to form the mesh prosthesis 100. Owing to the transparency of the film layer 3, the top surface 1b of the mesh layer 1 is visible from the back surface 5b of the mesh prosthesis 100.

The dimensions of the mesh prosthesis 100 (including the mesh layer 1 and the film layer 3) may range from 7.5 x 15 cm to 20 x 25 cm. In an embodiment, the dimension of the mesh prosthesis 100 is 15 x 15 cm.

The indicator 7 is provided on the top surface 3a of the film layer 3 of the mesh prosthesis 100. In an embodiment, the indicator 7 is placed at the center portion of the top surface 3a of the film layer 3 which may also be the center of the mesh prosthesis 100. The indicator 7 may be any symbol or an alphabet. In an embodiment, the indicator 7 is an 'F' shaped mark. As the film layer 3 is a transparent layer, the indicator 7 may be identified from the front surface 5a (FIG. 1) as well as the back surface 5b (FIG. 1a) of the mesh prosthesis 100. In an embodiment, as the indicator 7 is provided on the top surface 3a of the film layer 3, the indicator 7 is visualized in an inverted form from the front surface 5a of the mesh prosthesis 100 (shown in FIG. 1).

The indicator 7 may be made from the same material as that of the film layer 3.

The indicator 7 is provided on the top surface 3a of the film layer 3 to identify the visceral side of the mesh prosthesis 100. Further, the indicator 7 ensures ease of mesh orientation from the centre and helps the doctors during surgeries to avoid errors that are related to polypropylene side of mesh facing the organs. In an embodiment, the indicator 7 is colored so that it can be easily identified from the transparent film layer 3.

The indicator 7 may be attached to the top surface 3a of the film layer 3 using various techniques which may include without limitation, stamping, printing. In an embodiment, the indicator 7 is compressed over the film layer 3 (described in detail in Fig. 2). In an embodiment, the indicator 7 is prepared in the form of a thin film. The thin film may be prepared by the solvent casting method (described in detail in Fig. 2). In an embodiment, the same solution which is utilized for the formation of the film layer 3 i.e. PLCL (polymer) and dichloromethane

(solvent) is utilized for the formation of the thin film. The concentration of the polymer used for the formation of the indicator 7 may range from 2.0% to 4.0% (w/v).

The aforementioned solution used for the formation of the thin film may further include a coloring agent in order to provide a distinct color to the indicator 7. The coloring agent may include without limitation, D&C green, D&C orange, D&C red, D&C blue and D&C yellow. In an embodiment, the coloring agent used is D&C blue. The concentration of the coloring agent used in the aforementioned solution may range from 0.50% to 1.0% (w/v).

The thickness of the indicator 7 may range from 0.05- 0.08mm. In an embodiment, the thickness of the indicator 7 is 0.06mm. The dimensions of the indicator 7 may vary depending on the size of the mesh prosthesis 100. For example, for the mesh prosthesis 100 of size 15 x 15 cm, the length of the indicator 7 ranges from 15 mm to 20mm.

In an embodiment, the mesh prosthesis 100 (including the mesh layer 1, the film layer 3 and the indicator 7) may be coated with a therapeutic agent. The therapeutic agent may include any agent that may be used for relieving the pain that is caused to a patient post implantation of the mesh prosthesis 100. The therapeutic agent may include various categories including without limitation, anti-inflammatory agent, anti-bacterial agent, analgesic agent, antihistamines, steroidal or non-steroidal anti-inflammatory agents, matrix metalloproteinase (MMP) inhibitors, mucolytics, opioids, anti-proliferative agent, anticholinergics, antifungal agents, antiparasitic agents, antiviral agents, biostatic compositions, vasoconstrictors, and combinations thereof. In an embodiment, the therapeutic agent includes an analgesic agent.

The analgesic agent may include without limitation, non-steroidal anti-inflammatory drugs (NSAIDs), opioid analgesics, non-opioid analgesics and compound analgesics. The non-steroidal anti-inflammatory drug (NSAIDs) may include without limitation, ibuprofen, paracetamol, naproxen sodium, diclofenac sodium, celecoxib, rofecoxib, etoricoxib, indomethacin and naproxen or their combination. The opioids analgesics may include without limitation, codeine phosphate, tramadol hydrochloride, dextropropoxyphe hydrochloride and their derivatives. The steroidal anti-inflammatory analgesics may include without limitation, triamcinolone, hydrocortisone, mometasone, dexamethasone, betamethasone, amcinonide, desonide or their combination. The non-opioid analgesics may include without limitation, COX-2 inhibitors, capsaicin analogues, lumiracoxib, ketamine, pregabalin, duloxetine, tapentadol. The compound analgesics may include without limitation, co-codamol, phenacetin, caffeine, oxycodone and aspirin. Some other analgesics may include without limitation, leflunomide, sulfasalazine, methotrexate, hydroxychloroquine.

In an embodiment, the analgesic agent used is indomethacin. The said agent is widely used NSAID drug for treating pain, fever stiffness, swelling and inflammation. The dose of the analgesic agent used may range from 0.1 - 10µg/mm² more preferably, 1- 5µg/mm² more preferably, 3 - 5µg/mm².

Fig. 2 illustrates a flow chart depicting the manufacturing process of the mesh prosthesis 100.The said process commences at step 201.

At step 201, the polymeric solution (described above) is subjected to the solvent casting method in order to form the film layer 3. The said step may be performed using a substrate i.e. a vessel and/or container utilized for storing and casting the polymeric solution. The substrate utilized for the process of casting may be made of any material which includes without limitation, glass, teflon, teflon coated stainless steel and the like. The material selected for making the substrate must help in easy removal of the film layer 3 without damaging the said layer. In an embodiment, the substrate is a glass container wherein the glass is a high resistant type glass which can withstand high temperatures.

The polymeric solution is poured inside the glass substrate in order to commence the solvent casting method. The polymeric solution must be poured in a way that there is no inadvertent introduction of air bubbles. The substrate must be kept undisturbed over a horizontal even surface for 0.5 - 04 hours more preferably, for 01 to 02 hours at a predefined temperature of 20- 30°C more preferably, 22- 26°C.

At step 203, the substrate (including the casted polymeric solution) formed during the solvent casting method is subjected to primary annealing. The substrate formed during the solvent casting method may include a small amount of partially evaporated solvent. Therefore, the substrate is further subjected to heat treatment in order to completely evaporate the solvent and achieve uniformity in the film layer 3. The said step may be referred to as primary annealing. The step of primary annealing may be carried out at any temperature that is between the glass transition temperature and the melting temperature of the polymer utilized to form the film layer 3. In an embodiment, as the polymer used to form the film layer 3 is PLCL, the temperature for primary annealing may range from 50°C to 150°C more preferably, from 70°C to 90°C.

The step of primary annealing is performed when the substrate including the polymeric solution is loaded inside a vacuum oven ensuring that the polymeric solution is not disturbed inside the substrate. The time period for the step of primary annealing also acts as a significant parameter for heat treating of the polymeric solution. In an embodiment, the time period to carry out the step of primary annealing may range from 01 hour- 16 hours more preferably, 1.5 hours to 05 hours. The said step results in complete evaporation of the solvent in turn casting the polymeric solution into a thin film.

The primary annealed film layer 3 at this stage may be separated from the substrate for attachment with the mesh layer 1. In an embodiment, the thickness of the film layer 3 formed from the solvent casting method ranges from 0.05 - 0.08 mm. In an alternate embodiment, the primary annealed film layer 3 is kept inside the substrate for further processing. The substrate is further utilized as a protection for the casted film layer 3 to prevent adherence of foreign particles, wrinkling of the film layer 3 and ease of handling.

At step 205, the film layer 3 formed during the aforementioned step is subjected to adhesion and compression. In an embodiment, the top surface 3a of the film layer 3 is still integrated with the substrate while the bottom surface 3b is adhered to the mesh layer 1. The bottom surface 3b of the film layer 3 may be attached to the top surface 1b of the mesh layer 1 using various techniques known in the art. The techniques may include without limitation, sticking/adhesion, compression and the likewise or the combination of both techniques. In an embodiment, the film layer 3 is attached to the mesh layer 1 via a combination of both adhesion technique as well as compression.

In an embodiment, the first stage for attaching the top surface 1b of the mesh layer 1 and the bottom surface 3b of the film layer 3 is sticking the respective surface of the mesh layer 1 to the respective surface of the film layer 3. In order to stick the mesh layer 1 with the film layer 3, an adhesive agent is used. The adhesive agent may be any medical grade adhesive including without limitation, loctite 4014, dymax 213-CTH, etc.

In an alternate embodiment, a polymeric solution is used that permits sticking of the respective surfaces of the mesh layer 1 with the film layer 3. The polymer must be selected so that it is compatible with the film layer 3 PLCL and does not interfere with the degradation profile of the film layer 3. The polymer may be selected from the family of lactide and caprolactone. The examples of polymers may include without limitation, poly-L-lactide-co-caprolactone (PLCL), polycaprolactone (PCL), poly-dl-lactic acid (PDLLA), polyglycerolsebacate (PGS) or a mixture thereof. In an embodiment, the polymer used as an adhesive agent is PLCL.

The concentration of the solution of PLCL used may range from 1.0% to 10.0% (w/v) more preferably, 3.0% to 6.0% (w/v). The solvent used for the preparation of polymeric adhesive solution can be any organic solvent in which the polymer is completely soluble. In an embodiment, the solvent used is dichloromethane. The viscosity of solution may range from 0.1- 1.5dl/gm more preferably, 0.6 - 0.9 dl/gm. In an embodiment, the solution of PLCL may be subjected to ultra-sonication for a time period of 10 - 60 minutes to ensure complete and uniform solubility of PLCL in dichloromethane.

The adhesive may be applied between the mesh layer 1 and the film layer 3 using various methods known in the art including without limitation, spray coating, dip coating, manually and evenly spreading the solution over mesh, soaking the mesh with solution.

In an embodiment, the adhesive solution of PLCL prepared is poured into the glass substrate comprising the film layer 3 and further ensuring that no air bubbles are entrapped while pouring. The mesh layer 1 is then slowly set over the substrate that contains the adhesive solution of PLCL. The mesh layer 1 is set over the adhesive solution such that the top surface 1b of the mesh layer 1 is soaked with the adhesive solution of PLCL.

In the second stage of attachment of the respective surfaces of mesh layer 1 and the film layer 3, manual compression of the two surfaces of the mesh layer 1 and the film layer 3 is employed. The manual compression is performed such that even force is applied to the mesh layer 1 throughout its surface. The assembly of the mesh layer 1 and the film layer 3 (i.e. the mesh prosthesis 100) is left undisturbed at room temperature for drying. In an embodiment, a suitable weight is placed over the assembly of the mesh layer 1 and the film layer 3 to further compress the mesh layer 1 to facilitate adherence and ensure that the mesh is evenly attached to the solvent casted film layer 3. The time of drying and compression of the mesh layer 1 with the film layer 3 may range from 01hour to 24 hours, more preferably 06 hours to 12 hours.

At step 207, in order to ensure complete and uniform adherence of the mesh layer 1 with the film layer 3, the mesh layer 1 and the film layer 3 is subjected to a secondary annealing process. The substrate comprising of the mesh layer 1 and the film layer 3 is subjected to vacuum annealing in a vacuum oven. The secondary annealing is carried out at a temperature ranging from 60°C to 120°C more preferably, 80°C to 110°C. The time period for which the substrate is subjected to the secondary annealing process ranges from 01 hour to 10 hours more preferably, 02hours to 06 hours. The secondary annealing process relieves any internal stresses generated in the mesh prosthesis 100. The secondary annealing process also further enhances the strength of the film layer 3.

Further, the secondary annealed assembly of the mesh layer 1 and the film layer 3 is removed from the glass substrate by carefully peeling the top surface 3a of the film layer 3 off the substrate. The peeling is performed in manner that it does no damage to the edges of the film layer 3. The steps of primary and secondary annealing ensure firm attachment of the mesh layer 1 with the film layer 3 of the mesh prosthesis 100. The said steps also ensure uniform adhesion of the film layer 3 to the mesh layer 1 and the transparency of the film layer 3.

At step 209, the secondary annealed assembly of the mesh layer 1 and the film layer 3 is subjected to alcohol incubation process. The process of alcohol incubation may be carried out to remove excessive moisture of any kind that might be present in the secondary annealed assembly of the mesh layer 1 and the film layer 3. The said process involves immersion of the mesh layer 1 and the film layer 3 obtained in the above step of secondary annealing in an alcoholic solution. The said process ensures removal of moisture from the mesh layer 1 and the film layer 3 and in turn dries the said layers. The mesh prosthesis 100(including the mesh layer 1 and the film layer 3) which is alcohol incubated results in a completely dry prosthesis that easily unfolds at an implantation site during the deployment process. The mesh prosthesis 100 (including the mesh layer 1 and the film layer 3) must be completely dry as the surgeons fold the mesh prosthesis 100 in order to deploy it into the trocar for delivery at an implantation site wherein the mesh prosthesis 100 opens into its original configuration at the implantation site.

The process of alcohol incubation may utilize any type of alcohol that is acceptable for medical use including without limitation, methanol, ethanol, propanol, iso-propyl alcohol and the likewise. In an embodiment, the alcohol used to perform the process of alcohol incubation is ethanol. The concentration of alcohol used for the incubation process may range from 25% to 100% more preferably, 50% to 90%. The time period for performing the alcohol incubation process ranges from 01 hour to 24 hours more preferably, 06 hours to 10 hours.

At step 211, the mesh prosthesis 100 (including the mesh layer 1 and the film layer 3) is subjected to a process of formation of drainage holes 9. The drainage holes 9 are formed on the bottom surface 1a of the mesh layer 1 of the mesh prosthesis 100 (depicted in Fig. 1). The drainage holes 9 may be formed using various techniques. The various techniques may include without limitation, punching technique, laser technique, drilling and the likewise. In an embodiment, the drainage holes 9 are formed using laser technique. In an embodiment, the said technique is performed using a laser machine. The parameters for performing the laser technique include power and speed of the laser machine. The predefined power of the laser machine may be in the range of 03- 07% and the predefined speed at which the laser technique is performed may be in the range of 17 - 23%.

At step 213, the mesh prosthesis 100 (including the mesh layer 1 and the film layer 3) is subjected to the formation of the indicator 7. The indicator 7 is formed on the center portion of the top surface 3a of the film layer 3 of the mesh prosthesis 100. In an embodiment, the indicator 7 is prepared in the form of a thin film using the solvent casting method. In an embodiment, a thin film is prepared using a solution. The said solution includes a polymer, a solvent and a coloring agent (described above in Fig. 1). The said solution is poured into a glass substrate and kept undisturbed over a horizontal even surface for 0.5-4 hours more preferably, 01 to 02 hours. The said solution is kept at a predefined temperature of 20⁰C-30⁰C more preferably, 22°C-26°C.

The glass substrate with partially evaporated solvent is then subjected to a primary annealing process. The temperature for carrying out primary annealing process may range from 50°C to 150°C more preferably, 70°C to 90°C in a time period of 01 hour to 16 hours more preferably, 1.5 hours to 05 hours. Post the primary annealing process, the substrate with the said solution is loaded inside a vacuum oven. The aforesaid step results in complete evaporation of the solvent in turn casting the said solution into the thin colored film.

The thin colored film prepared using the solvent casting method is peeled from the glass substrate and is further utilized for preparing the indicator 7 which is provided in the form of for example, an 'F' shaped mark. The indicator 7 may be prepared from the casted film using any technique known in the art including manually cutting the film using a mold, laser cutting the film to achieve the mark and the likewise. In an embodiment, laser cutting technique is used for making the indicator 7 from the thin colored film. The thin colored film is subjected to the laser cutting process. In an embodiment, the said technique is performed using a laser machine. The parameters for performing the laser technique include power and speed of the laser machine. The predefined power of the laser machine may be in the range of 03- 07% and the predefined speed at which the laser technique is performed may be in the range of 17 - 23%.

Post the laser technique, the indicator 7 is adhered to the mesh prosthesis 100 (including the mesh layer 1 and the film layer 3) in order to provide the identification of the visceral side. The indicator 7 may be adhered to mesh prosthesis 100 through various methods namely attachment using adhesive agent, compression, combination of adhesion and compression techniques etc. In an embodiment, the laser cut indicator is compressed over the top surface 3a of the film layer 3 ensuring that the indicator 7 is located exactly in the centre of the mesh prosthesis 100 (as shown in Fig.1). After compression of the indicator 7 on the top surface 3a of the film layer 3, the mesh prosthesis 100 is kept under compression for a specific period of time ranging from 01 hour to 12 hours more preferably, 03 hours to 06 hours. The said step results into the formation of the mesh prosthesis 100 including the indicator 7 which helps in identifying visceral side of the mesh prosthesis 100 as well the orientation of the mesh prosthesis 100 from the centre portion of the said prosthesis 100.

At step 215, the mesh prosthesis 100 is subjected to packaging and sterilization. In an embodiment, the mesh prosthesis 100 is packaged in a PETG tray and sealed using the blister sealing technique. The mesh prosthesis 100 is further subjected to the sterilization process. The sterilization may be performed using gamma or e-beam radiation sterilization method. In an embodiment, the sterilization process is performed using gamma. The dose for gamma radiation process may range from 5 - 30kGy more preferably, 10 - 20kGy.

Post sterilization of the mesh prosthesis 100, the burst strength of the mesh prosthesis 100 was found to be in the range of 06- 15kgf. In an embodiment, the burst strength of prosthesis may be measured using an instrument called Ubique Burst Strength Tester. The instrument is a ball type plunger wherein the plunger ball is a metallic ball usually made up of stainless steel. The diameter of the plunger ball is approximately 25.44 mm ± 0.005 mm. In an embodiment, the mesh prosthesis 100 is clamped on a steel ring and steel mount. The clamping system may prevent pre-tensioning of the mesh prosthesis 100 before the test and slippage during the test. The ball type plunger is then advanced into and through the mesh prosthesis 100 at a constant rate of 300±13 mm/min. The push-through force (kgf) and push through displacement (mm) is measured. The push through force for the PLC coated mesh when the film thickness is in the range of 0.05 mm to 0.08mm is measured using this equipment. The push through force for the mesh prosthesis 100 prepared using the methods described in Fig. 2 is found to be in the range of 6- 15kgf more preferably, 8- 12kgf.

The mesh prosthesis 100 is also checked for the stickiness of the film layer 3 when folded in a form that the surgeons perform during the implantation process to pass through a trocar. During the implantation process, the mesh prosthesis 100 can be easily unfolded at the implantation site.

In-vitro simulated degradation study of the mesh prosthesis 100 was performed under physiological conditions with phosphate buffer saline (pH 7.4±0.2) at 77°C temperature. The accelerated in-vitro degradation study depicted gradual decrease in the molecular weight of the PLCL polymer throughout the study.

The present invention may further be understood by the following examples:
**Example 1:** Various concentrations of the polymeric solutions (including PLCL and dichloromethane) were prepared by dissolving the respective amount of PLCL in dichloromethane. The PLCL polymer is commercially available in the ratio of 70% lactide and 30% caprolactone. The said solution is sonicated for a period of 60 minutes to ensure complete dissolution of the polymer in the solvent. A specific volume of this solution is carefully poured inside the glass substrate ensuring that no air bubbles are entrapped. The substrate to be used is selected on the basis of the size and shape of the mesh layer 1. The mesh layer 1 used in this example is a rectangle mesh of size 7.5 x 15 cm. The substrate with polymeric solution is then allowed to remain undisturbed at room temperature for solvent evaporation. After complete solvent evaporation, the polymeric film is peeled off from the substrate. The film was then visually observed for strength, transparency and stickiness. The observations are provided below in Table 1:

**Table 1**

| **Sr. No.** | **Concentration of Polymeric Solution (%w/v)** | **Visual Observation** |
|---|---|---|
| 1 | 2.0 | Very thin film is formed hence it is very difficult to peel off from the substrate |
| 2 | 2.5 | |
| 3 | 3.0 | Film is formed; however it does not have sufficient strength to retain its shape. |
| 4 | 3.5 | |
| 5 | 4.0 | Film is formed but it does not have sufficient strength |

Hence it is noticed that even though the film layer 3 is formed it does not have enough strength to be adhered to the mesh layer 1.

**Example 2:** Various concentrations of polymeric solutions were prepared by dissolving PLCL in dichloromethane. The PLCL polymer is commercially available in the ratio of 70% lactide and 30% caprolactone. Similar method as mentioned in example 1 is followed, however in order to improve the strength of the film layer 3, the said layer is subjected to primary annealing at 90° C temperature for a period of 02 hours to 03 hours. The primary annealed film layer 3 is then peeled off from the glass substrate to visually check its structural integrity and measure its thickness using a thickness gauge. The measured thickness of the film layer 3 is provided below in table 2:

**Table 2**

| **Sr. No.** | **Concentration of Polymeric Solution (%w/v)** | **Visual Observation** | **Thickness (mm)** |
|---|---|---|---|
| 1 | 2.0 | Film is formed though difficult to peel off from the substrate | - |
| 2 | 2.5 | Film formed but difficult to separate from the substrate | - |
| 3 | 3.0 | Film is formed and easily separated from the substrate | 0.1-0.3 mm |
| 4 | 3.5 | Film is formed and easily separated from the substrate | 0.5-0.7 mm |
| 5 | 4.0 | Film is formed and separated from the substrate | 0.9-1.50 mm |

It is observed from the above table that the film casted from the solutions having concentration 3.0% to 4.0% have sufficient strength to get easily separated from the glass substrate. The optimum thickness of the film is achieved when the solution used for casting has a concentration of 3.0%to3.5% of PLCL.

**Example 3:** A PLCL solution of concentration 3.0% to 3.5% w/v is prepared and sonicated to obtain a uniform solution. The method mentioned in example 1 for solvent casting is used and the primary annealing of glass substrate is performed as mentioned in example 2. Further this solvent casted film is used for adhering the mesh layer 1. The mesh layer 1 of pore size ranging from 1.0mm to 2.0 mm is selected. An adhesion solution including of 4.0% to 5.0% PLCL(w/v) is taken in another glass substrate and the mesh layer 1 is soaked with the adhesive solution. The soaked the mesh layer 1 is then placed over the solvent casted film layer 3 such that the distal portion of the mesh layer 1 that is soaked with the adhesive solution comes in contact with the casted film layer 3. This polypropylene mesh-PLCL film assembly (i.e. the mesh prosthesis 100) is then subjected to compression by placing a weight over the assembly.

The above mentioned step results in uniform adherence of the film layer 3 with the mesh layer 1. Further, the mesh prosthesis 100 is subjected to secondary annealing at 80°C to 110°C temperature for 02 hours to 06 hours. Post-secondary annealing treatment, the film layer 3 is firmly adhered to the mesh layer 1 and is carefully peeled off from the glass substrate. The additional edges (if any) of the film layer 3 from the mesh prosthesis 100 is then is cut and separated. The mesh prosthesis 100 obtained after the aforesaid process is tested for its stickiness. The mesh prosthesis 100 is rolled to check if the mesh layer 1 sticks while folding. It is however observed that the rolled mesh prosthesis 100 does not immediately unfold.

**Example 4:** The mesh prosthesis 100 is manufactured using the same method as described above in example 3. However, in order to overcome the problems of the mesh prosthesis 100 achieved in example 3, the mesh prosthesis 100 is immersed in an alcoholic solution of concentration 50% to 90% for 06 hours to 10 hours. Post alcohol incubation the mesh prosthesis 100 is tested for the stickiness. It is observed that the ethanol incubated mesh prosthesis 100 easily unfolds as compared to the mesh prosthesis 100 achieved in example 3 (that is not treated with alcohol incubation). The alcohol incubated mesh prosthesis 100 is further packed in a Tyvek pouch and gamma sterilized at gamma radiation of 10kGy to 20kGy. The mesh prosthesis 100 is tested for film thickness. The film thickness of the film layer 3 irrespective of the alcohol incubation as measured on the edges is between 0.4mm to 0.6mm (provided below in table 3):

**Table 3**

| **S. No.** | **Treatment** | **Observation** | **Thickness (mm)** |
|---|---|---|---|
| 1 | PLCL Coated Hernia Mesh Without Alcohol Incubation | The prosthesis does not easily unfold and is slightly sticky | 0.4 to 0.6 |
| 2 | PLCL Coated Hernia Mesh With Alcohol Incubation | No stickiness if observed and the prosthesis unfolds without any difficulty | 0.4 to 0.6 |

## Claims

1. A method for manufacturing a mesh prosthesis (100), the method comprising:
a. solvent casting of a polymeric solution on a substrate, the polymeric solution consists of 3-3.5% of ploy-L-lactide-co-caprolactone dissolved in dichloromethane;
b. primary annealing of the substrate including the casted polymeric solution to evaporate a partial amount of dichloromethane from the casted polymeric solution, wherein the primary annealing is performed to form a transparent film layer (3) over the substrate having a top surface (3a) and a bottom surface (3b);
c. providing a mesh layer (1) having a top surface (1b) and a bottom surface (1a);
d. placing the bottom surface (3b) of the transparent film layer (3) in close contact with the top surface (1b) of the mesh layer (1) and compressing the transparent film layer (3) and the mesh layer (1) to form a mesh prosthesis (100) having a front surface (5a);
e. secondary annealing of the mesh prosthesis (100) to ensure complete and uniform adhesion and compression of the transparent film layer (3) and the mesh layer (1);
f. removing the substrate from the annealed mesh prosthesis (100);
g. incubating the mesh prosthesis (100) of step e with an alcohol to remove excessive moisture;
h. forming one or more drainage holes (9) on the bottom surface (1a) of the mesh layer (1) of the mesh prosthesis (100) to facilitate fibroblast growth and to enable drainage of seroma, the drainage holes having a diameter in the range of 0.5-1.25mm;
i. forming an indicator (7) on a centre portion of the top surface (3a) of the transparent film layer (3) of the mesh prosthesis (100) to identify the visceral side of the mesh prosthesis (100), wherein the indicator being visible in an inverted form from the front surface (5a) of the mesh prosthesis (100) via the transparent film layer (3) thereby providing ease in mesh orientation and avoiding errors relating to the orientation of the mesh prosthesis (100); and
j. packaging and sterilizing the mesh prosthesis (100).

2. The method for manufacturing the mesh prosthesis (100) as claimed in claim 1, wherein the primary annealing of the casted polymeric solution is performed at a temperature ranging from 50°C to 150°C for a time period ranging from 01 hour- 16hours.

3. The method for manufacturing the mesh prosthesis (100) as claimed in claim 1, wherein the placing comprises applying an adhesive agent and/or a polymer between the transparent film layer (3) and the mesh layer (1).

4. The method for manufacturing the mesh prosthesis (100) as claimed in claim 1, wherein the secondary annealing of the transparent film layer (3) and the mesh layer (1) assembly includes annealing at a temperature ranging from 60°C to 120°C for a time period ranging from 01 hour to 10 hours.

5. The method for manufacturing the mesh prosthesis (100) as claimed in claim 1, wherein the alcohol is selected from methanol, ethanol, propanol, and iso-propyl alcohol.

6. The method for manufacturing the mesh prosthesis (100) as claimed in claim 1, wherein concentration of the alcohol ranges from 25% to 100%.

7. The method for manufacturing the mesh prosthesis (100) as claimed in claim 1, wherein the incubating comprises incubating the annealed mesh prosthesis for a time period ranging from 01 hour to 24 hours.

## Patentansprüche

1. Verfahren zur Herstellung einer Netzprothese (100), wobei das Verfahren Folgendes umfasst:
a. Lösungsmittelgießen einer Polymerlösung auf ein Substrat, wobei die Polymerlösung aus 3-3,5% Poly-L-lactid-co-caprolacton, gelöst in Dichlormethan, besteht;
b. Erstglühen des Substrats, einschließlich der gegossenen Polymerlösung, um eine Teilmenge des Dichlormethans aus der gegossenen Polymerlösung zu verdampfen, wobei das Erstglühen zur Bildung einer transparenten Folienschicht (3) über dem Substrat durchgeführt wird, die eine Oberseite (3a) und eine Unterseite (3b) aufweist;
c. Bereitstellen einer Netzschicht (1), die eine Oberseite (1b) und eine Unterseite (1a) aufweist;
d. Platzieren der Unterseite (3b) der transparenten Folienschicht (3) in engem Kontakt mit der Oberseite (1b) der Netzschicht (1) und Komprimieren der transparenten Folienschicht (3) und der Netzschicht (1), um eine Netzprothese (100) zu bilden, die eine Vorderseite (5a) aufweist;
e. Zweitglühen der Netzprothese (100), um eine vollständige und gleichmäßige Haftung und Kompression der transparenten Folienschicht (3) und der Netzschicht (1) zu gewährleisten;
f. Entfernen des Substrats von der geglühten Netzprothese (100);
g. Inkubieren der Netzprothese (100) aus Schritt e mit Alkohol, um überschüssige Feuchtigkeit zu entfernen;
h. Ausbilden eines oder mehrerer Drainagelöcher (9) auf der Unterseite (1a) der Netzschicht (1) der Netzprothese (100) zur Förderung des Fibroblastenwachstums und zur Ermöglichung des Abflusses von Seromen, wobei die Drainagelöcher einen Durchmesser im Bereich von 0,5-1,25 mm aufweisen;
i. Bilden eines Indikators (7) in einem mittleren Abschnitt der Oberseite (3a) der transparenten Folienschicht (3) der Netzprothese (100) zur Kennzeichnung der viszeralen Seite der Netzprothese (100), wobei der Indikator von der Vorderseite (5a) der Netzprothese (100) durch die transparente Folienschicht (3) in umgekehrter Form sichtbar ist, wodurch die Ausrichtung des Netzes erleichtert und Fehler in Bezug auf die Ausrichtung der Netzprothese (100) vermieden werden; und
j. Verpacken und Sterilisieren der Netzprothese (100).

2. Verfahren zur Herstellung der Netzprothese (100) nach Anspruch 1, wobei das Erstglühen der gegossenen Polymerlösung bei einer Temperatur im Bereich von 50 °C bis 150 °C über einen Zeitraum im Bereich von 01 Stunde bis 16 Stunden erfolgt.

3. Verfahren zur Herstellung der Netzprothese (100) nach Anspruch 1, wobei das Platzieren das Auftragen eines Klebstoffs und/oder eines Polymers zwischen der transparenten Folienschicht (3) und der Netzschicht (1) umfasst.

4. Verfahren zur Herstellung der Netzprothese (100) nach Anspruch 1, wobei das Zweitglühen der Anordnung aus transparenter Folienschicht (3) und Netzschicht (1) das Glühen bei einer Temperatur im Bereich von 60°C bis 120°C über einen Zeitraum im Bereich von 01 Stunde bis 10 Stunden einschließt.

5. Verfahren zur Herstellung der Netzprothese (100) nach Anspruch 1, wobei der Alkohol aus Methanol, Ethanol, Propanol und Isopropylalkohol ausgewählt ist.

6. Verfahren zur Herstellung der Netzprothese (100) nach Anspruch 1, wobei die Alkoholkonzentration im Bereich von 25 % bis 100% liegt.

7. Verfahren zur Herstellung der Netzprothese (100) nach Anspruch 1, wobei das Inkubieren das Inkubieren der geglühten Netzprothese über einen Zeitraum im Bereich von 01 Stunde bis 24 Stunden umfasst.

## Revendications

1. Procédé de fabrication d'une prothèse à mailles (100), le procédé comprenant :
a. une coulée de solvant d'une solution polymère sur un substrat, la solution polymère est constituée de 3 à 3,5 % de poly-L-lactide-co-caprolactone dissous dans du dichlorométhane ;
b. un recuit primaire du substrat incluant la solution polymère coulée pour évaporer une quantité partielle de dichlorométhane de la solution polymère coulée, dans lequel le recuit primaire est effectué pour former une couche de film transparent (3) sur le substrat présentant une surface supérieure (3a) et une surface inférieure (3b) ;
c. une fourniture d'une couche de mailles (1) présentant une surface supérieure (1b) et une surface inférieure (1a) ;
d. une mise en place de la surface inférieure (3b) de la couche de film transparent (3) en contact étroit avec la surface supérieure (1b) de la couche de mailles (1) et une compression de la couche de film transparent (3) et la couche de mailles (1) pour former une prothèse à mailles (100) présentant une surface avant (5a) ;
e. un recuit secondaire de la prothèse à mailles (100) pour assurer une adhésion et une compression complètes et uniformes de la couche de film transparent (3) et de la couche de mailles (1) ;
f. un retrait du substrat de la prothèse à mailles recuite (100) ;
g. une incubation de la prothèse à mailles (100) de l'étape e avec de l'alcool pour éliminer l'excédent d'humidité ;
h. une formation d'un ou plusieurs trous de drainage (9) sur la surface inférieure (1a) de la couche de mailles (1) de la prothèse à mailles (100) pour faciliter la croissance des fibroblastes et permettre le drainage du sérome, les trous de drainage présentant un diamètre dans la plage de 0,5 à 1,25 mm ;
i. une formation d'un indicateur (7) sur une partie centrale de la surface supérieure (3a) de la couche de film transparent (3) de la prothèse à mailles (100) afin d'identifier le côté viscéral de la prothèse à mailles (100), dans lequel l'indicateur est visible sous forme inversée depuis la surface avant (5a) de la prothèse à mailles (100) à travers la couche de film transparent (3), en facilitant ainsi l'orientation de mailles et en évitant des erreurs relatives à l'orientation de la prothèse à mailles (100) ; et
j. un emballage et une stérilisation de la prothèse à mailles (100).

2. Procédé de fabrication de la prothèse à mailles (100) selon la revendication 1, dans lequel le recuit primaire de la solution polymère coulée est effectué à une température dans la plage de 50°C à 150°C pendant une période dans la plage de 1 heure à 16 heures.

3. Procédé de fabrication de la prothèse à mailles (100) selon la revendication 1, dans lequel la mise en place comprend l'application d'un agent adhésif et/ou un polymère entre la couche de film transparent (3) et la couche de mailles (1).

4. Procédé de fabrication de la prothèse à mailles (100) selon la revendication 1, dans lequel le recuit secondaire de l'ensemble de couche de film transparent (3) et de couche de mailles (1) inclut un recuit à une température dans la plage de 60°C à 120°C pendant une durée dans la plage de 1 heure à 10 heures.

5. Procédé de fabrication de la prothèse à mailles (100) selon la revendication 1, dans lequel l'alcool est choisi parmi le méthanol, l'éthanol, le propanol et l'alcool isopropylique.

6. Procédé de fabrication de la prothèse à mailles (100) selon la revendication 1, dans lequel la concentration d'alcool est dans la plage de 25 % à 100 %.

7. Procédé de fabrication de la prothèse à mailles (100) selon la revendication 1, dans lequel l'incubation comprend l'incubation de la prothèse à mailles recuite pendant une période dans la plage de 1 heure à 24 heures.
